(19) Europäisches Patentamt
European Patent Office
Office européen des brevets

(11) Veröffentlichungsnummer: **0 012 850**
**B1**

(12) # EUROPÄISCHE PATENTSCHRIFT

(45) Veröffentlichungstag der Patentschrift: **25.11.81**

(51) Int. Cl.³: **C 07 C 45/45,** C 07 C 49/00 // C07C69/76, C07C79/36, C07D307/46, C07D333/22

(21) Anmeldenummer: **79104630.3**

(22) Anmeldetag: **21.11.79**

(54) **Verfahren zur Herstellung von Ketonen durch Umsetzung von Carbonsäurehalogeniden mit Alkylaluminiumverbindungen.**

(30) Priorität: **28.11.78 DE 2851371**
**12.10.79 DE 2941391**

(43) Veröffentlichungstag der Anmeldung:
**09.07.80 Patentblatt 80/14**

(45) Bekanntmachung des Hinweises auf die Patenterteilung:
**25.11.81 Patentblatt 81/47**

(84) Benannte Vertragsstaaten:
**AT BE CH DE FR GB IT NL**

(56) Entgegenhaltungen:
**DE-B-1 249 849**
**CHEMICAL ABSTRACTS, Vol. 59, 1963 Columbus, Ohio, USA Seite 8640e**
**CHEMICAL ABSTRACTS, Vol. 59, 1963 Columbus, Ohio, USA Seite 8640d**
**CHEMICAL ABSTRACTS, Vol. 60, 1964 Columbus, Ohio, USA Seite 14 420c**
**TENSIDE, Bd. 4, Nr.6, 1967, München, CARL HANSER ZEITSCHRIFTENVERLAG GMBH, H. REINHECKEL und R. HAAGE »Über Reaktionen mit Aluminiumalkylen, VIII«, Seiten 167 bis 171**

(73) Patentinhaber: **HOECHST AKTIENGESELLSCHAFT, Postfach 80 03 20, D-6230 Frankfurt/Main 80 (DE)**

(72) Erfinder: **Spielmann, Werner, Dr., Johann-Strauss-Strasse 18a, D-6233 Kelkheim (Taunus) (DE)**
Erfinder: **Schaeffer, Georg, Dr., Herderstrasse 58, D-6238 Hofheim am Taunus (DE)**

EP 0 012 850 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

# Verfahren zur Herstellung von Ketonen durch Umsetzung von Carbonsäurehalogeniden mit Alkylaluminiumverbindungen

Ketone sind wertvolle Zwischen- und Endprodukte auf zahlreichen wichtigen Fachgebieten, z. B. auf dem Gebiet der Farbstoffe, der Pflanzenschutzmittel, der Pharmazeutika und Lösungsmittel.

Zur Herstellung von Ketonen ist eine Reihe von klassischen Methoden bekannt. Eine neuere Methode geht von Carbonsäurehalogeniden aus, welche mit aluminiumorganischen Verbindungen (Al-alkyle und Alkyl-Al-halogenide) zu den jeweiligen Ketonen umgesetzt werden.

Als Lösungsmittel für diese Umsetzung hat man zunächst Benzol verwendet, worin man die Reaktion bei Temperaturen bis zu 80°C durchführte [J. Am. Chem. Soc. 73, 2854—56 (1951)]. Für diese Methode sind zwar ziemlich hohe Ketonausbeuten angegeben, doch läßt es sich hier nicht vermeiden, daß infolge der katalytischen Wirkung der verwendeten Al-Verbindungen auch Friedel-Crafts-Acylierungen des Lösungsmittels Benzol stattfinden, wodurch natürlich nicht unerhebliche Ausbeuteverluste eintreten und sich u. U. Schwierigkeiten bei der Abtrennung und Reinigung des gewünschten Ketons ergeben können.

Zur Vermeidung von Friedel-Crafts-Acylierungen des Lösungsmittels hat man das Lösungsmittel Benzol dann durch aliphatische Kohlenwasserstoffe (Pentan, Hexan, etc.) ersetzt [Fette-Seifen-Anstrichmittel 64, 881—86 (1962)]. Die Reaktionstemperatur soll hier aber nicht wesentlich höher als 0°C liegen, da andernfalls infolge vermehrter Bildung von Nebenprodukten die Ausbeute an dem gewünschten Keton absinken soll.

Bei der Erprobung weiterer, möglichst noch günstigerer Lösungsmittel hat man schließlich als »Lösungsmittel der Wahl« das Methylenchlorid $CH_2Cl_2$ gefunden [Tenside 4, 167—71 (1967); Organometalic Chemical Reviews A, S. 47—136, insbesondere S. 55/56 (1968); Houben Weyl, Bd. VII/2a 4. Aufl. Ketone Teil I, S. 573—575 (1973)]. Dieses Lösungsmittel besitzt gegenüber den vorher als Lösungsmittel verwendeten aromatischen und aliphatischen Kohlenwasserstoffen den Vorteil, daß darin praktisch alle Reaktionspartner und Endprodukte löslich sind. Die Reaktionstemperatur soll auch bei dieser Verfahrensweise nicht höher als etwa 0°C — möglichst jedoch darunter — sein, da — wie in der einschlägigen Literatur mehrfach deutlich hervorgehoben ist — bei Temperaturen über etwa 0°C unerwünschte Nebenreaktionen immer stärker zunehmen und damit natürlich die Ausbeuten an den gewünschten Ketonen immer weiter abnehmen sollen.

In der Zeitschrift Tenside 4, 167—71 (1967) wird zwar bei der Umsetzung von Capronylchlorid mit Triäthylaluminium im Molverhältnis 3 : 1 in Methylenchlorid bei +25°C von einer höheren Ausbeute an Octanon-(3) berichtet (39% d. Th.) als bei —30°C (Ausbeute 26% d. Th.), doch ist die höhere Ausbeute bei der höheren Temperatur hier allein durch die bei der höheren Temperatur verstärkte Dissoziation der bei tieferen Temperaturen recht stabilen Komplexverbindung aus dem bei der Reaktion entstehenden Keton und dem aus dem Triäthylaluminium zunächst entstehenden Diäthylaluminiumchlorid bedingt. Es heißt nämlich im Zusammenhang mit dieser Reaktion ausdrücklich, daß bei der höheren Temperatur die Nebenreaktionen verstärkt stattfinden.

Dies steht auch im Einklang mit den in dieser Literaturstelle ebenfalls beschriebenen Ergebnissen der Umsetzung von n-Butyrylchlorid mit n-Hexylaluminiumdichlorid im Molverhältnis 1 : 1 in Methylenchlorid zu Decanon-(4) bei —30 und bei +25°C. Bei —30°C wird eine Ausbeute an Decanon-(4) von 82% d. Th., bei 25°C eine Ausbeute von 71% d. Th. angegeben. Auch hier heißt es ausdrücklich, daß bei der höheren Temperatur die Menge der gebildeten Nebenprodukte zunimmt. Da die bei dieser Reaktion gebildete Komplexverbindung aus Octanon-(4) und Aluminiumchlorid kein zu einer weiteren Umsetzung mit Ausgangs-Säurechlorid befähigtes Alkylaluminiumhalogenid mehr enthält, kann hier die eigentliche Ketonbildungsreaktion durch eine bei erhöhter Temperatur verstärkte Komplex-Dissoziation nicht beeinflußt werden.

Nach dem einschlägigen Stand der Technik bestand also — sofern man keine beträchtliche Ausbeuteverminderung in Kauf nehmen wollte — ein klares Vorurteil dagegen, die bekannte Ketonsynthese aus Carbonsäurehalogeniden und Al-organischen Verbindungen in $CH_2Cl_2$ bei Temperaturen merklich bzw. erheblich über etwa 0°C durchzuführen. Andererseits muß aber zwecks Einhaltung der erforderlichen tiefen Reaktionstemperaturen wegen der exothermen Umsetzung ein nicht unerheblicher Kühlungsaufwand getrieben werden. Wegen des ansonsten guten Verlaufs und der guten Ausbeuten sowie der Möglichkeit, nach dieser Methode auf andere Weise nicht oder schwer erhältliche Ketone herstellen zu können, war es daher wünschenswert und bestand die Aufgabe, die Methode noch zu verbessern und möglichst wirtschaftlicher zu gestalten.

Diese Aufgabe konnte erfindungsgemäß in einfacher und ausgezeichneter Weise dadurch gelöst werden, daß man die fragliche Umsetzung bei Temperaturen, bei welchen die Wärmeabfuhr wesentlich wirtschaftlicher vorgenommen werden kann, d. h. im Bereich von 30 bis 60°C, durchführt. Überraschenderweise treten die von der einschlägigen Literatur vorhergesagten verschlechterten Ausbeuten (gegenüber der Durchführungsweise bei Temperaturen um oder unter 0°C) nicht — oder jedenfalls nicht in einem ins Gewicht fallenden Ausmaß — ein. In vielen Fällen wurde sogar eine erhebliche Ausbeutesteigerung festgestellt. Die Erfindung hat somit ein klares technisches Vorurteil überwunden und gleichzeitig ein bekanntes vorteilhaftes Verfahren noch vorteilhafter und wirtschaftlicher gemacht.

Erfindungsgegenstand ist nun ein Verfahren zur Herstellung von Ketonen durch Umsetzung von Carbonsäurehalogeniden mit Al-Alkylen oder Alkyl-Al-halogeniden, gegebenenfalls in Gegenwart eines Aluminiumhalogenids, in $CH_2Cl_2$ als Lösungsmittel und Zersetzung des entstehenden Ketonkomplexes mit Wasser, das dadurch gekennzeichnet ist, daß man die Umsetzung bei einer Temperatur im Bereich von 30 bis 60°C und insbesondere etwa 40°C — der Rückflußtemperatur des siedenden $CH_2Cl_2$ — gegebenenfalls unter Anwendung von Überdruck, durchführt.

Das Verfahren eignet sich zur Herstellung aller möglichen Ketone. Voraussetzung ist lediglich, daß die Ausgangs-Carbonsäurehalogenide keine weiteren funktionellen Gruppen besitzen, welche mit Al-Alkylen oder Alkyl-Al-halogeniden in unerwünschter Weise reagieren können. Ansonsten können die Carbonsäurehalogenide aliphatischer, aromatischer, araliphatischer oder heterocyclischer Natur sein und sowohl eine oder mehrere Säurehalogenidgruppen besitzen. Bevorzugte Carbonsäurehalogenide sind solche, welche von der allgemeinen Formel

$$R^1COX$$

umfaßt werden, in welcher

$R^1$ = a) ein gesättigter oder ungesättigter, verzweigter oder unverzweigter aliphatischer Rest, vorzugsweise mit 4 bis 20, insbesondere mit 4 bis 8 C-Atomen;

b) ein gegebenenfalls ein- oder mehrfach substituierter Aryl-, vorzugsweise Phenylrest; wenn der Aryl- bzw. Phenylrest substituiert ist, ist er dies vorzugsweise 1- oder 2mal, und zwar hauptsächlich durch $C_1-C_4$-Alkylreste — insbesondere $CH_3$-, Alkoxy-, insbes. $C_1-C_4$-Alkoxy-reste, Aryloxy-, insbes. Phenoxy-reste (ggf. 1—2 subst. wie obiger Arylrest), Halogen, insbesondere Cl, Br, $-NO_2$, COOR′ (R′ = $C_1-C_4$-Alkyl, vorzugsweise $CH_3$), Sulfamoyl $SO_2NH_2$ oder $SO_2NR''R'''$ (R″ und R‴ = organische Reste).

c) ein Aralkylrest, dessen aromatischer Teil vorzugsweise ein Phenylrest ist und in gleicher Weise 1- oder mehrfach substituiert sein kann wie der unter b) genannte Arylrest, und dessen aliphatischer Teil vorzugsweise 1—3 C-Atome besitzt, oder

d) ein heterocyclischer — vorzugsweise O- und/oder S-haltiger heterocyclischer — Rest, insbesondere Furyl- oder Thienylrest, und

X = Halogen (Cl, Br, J), vorzugsweise Cl.

Als konkrete Ausgangs-Carbonsäurehalogenide seien in beispielhafter Weise genannt:

n-Butyrylchlorid
n-Octadecansäurechlorid
3,3-Dimethylacrylsäurechlorid
Benzoylchlorid
3-Methoxybenzoylchlorid
3-Phenoxybenzoylchlorid
o-Chlorbenzoylchlorid
p-Chlorbenzoylchlorid
3-Chlor-5-methylbenzoylchlorid
2,6-Dichlorbenzoylchlorid
m-Brombenzoylchlorid
p-Brombenzoylchlorid
p-Methylbenzoylchlorid
p-tert.-Butyl-benzoylchlorid
p-Nitrobenzoylchlorid
p-Carbomethoxybenzoylchlorid
m-Carbobutoxybenzoylchlorid
Phenylessigsäurechlorid
4-Chlorphenylessigsäurechlorid
Zimtsäurechlorid
4-Chlorzimtsäurechlorid
Furan-2-carbonsäurechlorid
Thiophen-2-carbonsäurechlorid.

Für die Umsetzung mit den Carbonsäurehalogeniden nach dem erfindungsgemäßen Verfahren werden Al-Alkyle und Alkyl-Al-halogenide der allgemeinen Formel

$$R^2_nAlX_{3-n}$$

verwendet, worin

$R^2$ = gesättigter verzweigter oder unverzweigter Alkylrest, vorzugsweise mit 1 bis 12, insbesondere mit 1 bis 3 C-Atomen;
X = Halogen (Cl, Br, J), vorzugsweise Cl und
n = 1, 1,5, 2 oder 3, vorzugsweise 1,5 (=Al-sesqui-Halogenide).

Unter diese Formel fallende konkrete Verbindungen sind z. B. Methylaluminiumdichlorid, Methylaluminiumsesquichlorid, Dimethylaluminiumchlorid, Trimethylaluminium, Äthylaluminiumsesquichlorid, Äthylaluminiumsesqui-jodid, Tri-n-propyl-aluminium, Tri-n-hexyl-aluminium, n-Hexylaluminiumdichlorid und n-Dodecyl-aluminiumdibromid.

Wenn bei dem Verfahren zusätzlich zu den Al-Alkylen oder Alkyl-Al-halogeniden noch ein Aluminiumhalogenid verwendet wird, ist es bevorzugt, ein Al-Halogenid mit dem gleichen Halogenrest wie das Alkyl-Al-halogenid einzusetzen. Da als Alkyl-Al-halogenide Chloride bevorzugt sind, ist als Aluminiumhalogenid auch $AlCl_3$ bevorzugt.

Die Verwendung eines Aluminiumhalogenids zusätzlich zu dem jeweiligen Al-Alkyl oder Alkyl-Al-halogenid geschieht mit dem Zweck, möglichst alle Alkylgruppen des Al-Alkyls oder Alkyl-Al-halogenids für die Umsetzung mit dem entsprechenden Carbonsäurehalogenid auszunutzen. Die bei dem Verfahren gebildeten Ketone bilden nämlich mit den Al-Alkylen und Alkyl-Al-halogeniden ziemlich stabile Komplexe, wodurch dann die Alkylgruppen der Al-organischen Verbindungen für die weitere Umsetzung mit dem Carbonsäurehalogenid nicht mehr zur Verfügung stehen. Da Al-Halogenide wie etwa $AlCl_3$ stärkere Lewis-Säuren sind als Al-Alkyle und Alkyl-Al-halogenide, verdrängen sie diese aus den Komplexen und machen sie daher für die weitere Umsetzung mit den Carbonsäurehalogeniden verfügbar. Daher ist es bevorzugt, pro Äquivalent Carbonsäurehalogenid (ein Äquivalent eines nur eine COX-Gruppe enthaltenden Carbonsäurehalogenids = 1 Mol)

a) etwa 1 Mol Alkyl-Al-dihalogenid $R^2AlX_2$ (und kein Al-Halogenid)
b) etwa $^1/_2$ Mol Dialkyl-Al-halogenid $R^2_2AlX$ + etwa $^1/_2$ Mol Al-Halogenid
c) etwa $^2/_3$ Mol Alkyl-Al-sesqui-Halogenid $R^2_{1,5}AlX_{1,5}$ + etwa $^1/_3$ Mol Al-Halogenid oder
d) etwa $^1/_3$ Mol Trialkyl-Al $R^2_3Al$ + etwa $^2/_3$ Mol Al-Halogenid

zu verwenden. Dabei ist es günstig, wenn die Al-organische Verbindung in etwa 5%igem Überschuß eingesetzt wird. Höhere Überschüsse bringen keinen Vorteil. Das gleiche gilt im Prinzip auch für das Al-Halogenid.

Besitzen die Carbonsäurehalogenide solche Substituenten (wie z. B. Ketogruppen), die mit Al-organischen Verbindungen stabile Komplexe bilden, so werden pro Substituent ein Äquivalent an Al-organischer Verbindung oder an Al-Halogenid zusätzlich benötigt.

Das Verfahren wird zweckmäßig so durchgeführt, daß man $CH_2Cl_2$ vorlegt und darin das Al-Halogenid — falls ein solches erforderlich ist — suspendiert. Anschließend wird unter sorgfältigem Sauerstoffausschluß die für den Ansatz berechnete Menge Al-Alkyl oder Alkyl-Al-halogenid zugefügt. In diese Mischung läßt man das Säurechlorid so schnell einfließen, daß der einsetzende Methylenchlorid-Rückfluß leicht unter Kontrolle gehalten werden kann. In diesem Fall beträgt die Reaktionstemperatur etwa 40° C. Nach beendeter Zugabe des Säurechlorids wird noch kurze Zeit — im allgemeinen etwa 1 Stunde — nachgerührt.

Wenn man das Säurechlorid langsamer zufließen läßt, reicht die bei der exothermen Reaktion entstehende Wärme unter Umständen nicht aus, um das $CH_2Cl_2$ zum Sieden zu bringen. In diesem Falle liegt die Reaktionstemperatur dann unter etwa 40° C im Bereich von 30° und etwa 40° C.

In manchen Fällen kann es auch zweckmäßig sein, die Reaktion bei Temperaturen über etwa 40° C durchzuführen. Es muß dann allerdings unter Überdruck gearbeitet werden, wobei die Verwendung eines geschlossenen Gefäßes, in welchem sich bei der höheren Temperatur der entsprechende autogene Druck von selbst einstellt, sinnvoll ist. Die günstigste Durchführungsweise des Verfahrens ist jedoch im allgemeinen diejenige unter Normaldruck bei der Rückflußtemperatur des $CH_2Cl_2$ (etwa 40° C).

Nach beendeter Reaktion wird das Reaktionsgemisch zwecks Zersetzung der entstandenen Keton-Al-Halogenid-Komplexe mit Wasser versetzt, wozu man das Reaktionsgemisch zweckmäßig auf Wasser oder Eis fließen läßt. Wegen der dabei entstehenden Wärme beginnt hier das $CH_2Cl_2$ normalerweise zu sieden und kann dann abdestilliert oder unter Rückfluß gehalten werden. Aus dem mit Wasser behandelten Reaktionsansatz wird dann das gewünschte Keton auf übliche Weise, z. B. durch Destillation, gewonnen.

Nach dem Verfahren erhältliche bzw. erhaltene Ketone sind beispielsweise:

Hexanon-3
Decanon-4
Nonadecan-2-on
Mesityloxid

Butyrophenon
Propiophenon
3-Methoxyacetophenon
3-Phenoxyacetophenon
o-Chloracetophenon
o-Chlorpropiophenon
m-Chloracetophenon
3-Chlor-5-methylpropiophenon
2,6-Dichloracetophenon
m-Brompropiophenon
p-Bromacetophenon
p-Brompropiophenon
p-Methylacetophenon
p-Methylpropiophenon
p-tert.-Butyl-butyrophenon
p-Nitroacetophenon
p-Carbomethoxyacetophenon
m-Carbobutoxy-butyrophenon
Phenylaceton
1-Phenylbutanon-2
4-Chlorphenylacetophenon
4-Chlorbenzalaceton
2-Acetylthiophen
2-Acetylfuran.

Die Ketonausbeute ist bei diesem Verfahren durchweg mindestens etwa genauso hoch wie bei der herkömmlichen Verfahrensweise, bei welcher (in $CH_2Cl_2$) nur bei Temperaturen um oder — vorzugsweise — unter 0° C gearbeitet wurde. In einigen Fällen können wirtschaftliche Ausbeuten sogar nur bei der höheren Reaktionstemperatur erreicht werden. Dies war aufgrund der Aussagen in der anfangs erwähnten einschlägigen Literatur in keiner Weise zu erwarten und stellt darüber hinaus vor allem wegen des Wegfalls der Notwendigkeit, den Reaktionsansatz durch Kühlung um oder unter 0° C zu halten, eine sehr erhebliche betriebliche Verbesserung dar. Im einzelnen können die für den Fortschritt des Verfahrens maßgebenden Gründe wie folgt spezifiziert werden:

a) Durch das Arbeiten bei höheren Temperaturen, speziell in siedendem Methylenchlorid bei 40° C drucklos oder darüber bei Überdruck, werden aufwendige Kühlsysteme vermieden und es kann die verfahrenstechnisch äußerst einfach zu kontrollierende Verdampfungskühlung durch Rückflußsieden des Lösungsmittels ausgenutzt werden.
b) Reaktionsträge Carbonsäurechloride lassen sich bei der höheren Temperatur in kürzerer Reaktionszeit umsetzen.
c) Bei der höheren Temperatur besteht kaum die Gefahr, daß Verzögerungen bei der Reaktion zu sicherheitstechnisch bedenklichen Konzentrationen an unumgesetzten Reaktanten führen.

Die Erfindung wird nun durch die folgenden Beispiele näher erläutert. Falls nicht anders angegeben wurden sämtliche Erfindungsbeispiele nach folgender Verfahrensweise durchgeführt:

Es wurde $CH_2Cl_2$ vorgelegt und darin festes $AlCl_3$ (wasserfrei) suspendiert. Anschließend wurde das Alkyl-Al oder Alkyl-Al-halogenid zugefügt. In diese Mischung wurde das jeweilige Carbonsäurechlorid so schnell einfließen gelassen, daß der einsetzende $CH_2Cl_2$-Rückfluß unter Kontrolle gehalten werden konnte. Nach beendeter Zugabe wurde 1 Stunde nachgerührt.

Das Reaktionsgemisch ließ man anschließend auf Wasser fließen, wobei durch die exotherme Zersetzungsreaktion das Methylenchlorid zum Sieden kam. Der Ansatz wurde dann durch Destillation weiter aufgearbeitet.

Wenn nicht anders angegeben, wurden jeweils 0,3 Mol Carbonsäurechlorid mit 0,1 Mol $AlCl_3$ und 0,21 Mol $(CH_3)_{1,5}AlCl_{1,5}$ bzw. $(C_2H_5)_{1,5}AlCl_{1,5}$ in ca. 1 bis 10 g $CH_2Cl_2$ pro g Carbonsäurechlorid umgesetzt.

Die in die folgende Tabelle neben den Erfindungsbeispielen aufgenommenen Vergleichsbeispiele wurden in gleicher Weise durchgeführt, nur daß der Reaktionsansatz durch Außenkühlung immer auf einer Temperatur um oder unter 0° C gehalten wurde. Die Ergebnisse sind tabellarisch zusammengestellt.

Tabelle

| | Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|---|
| A) aliphatisch: | $CH_3(CH_3)_2COCl$ | $(C_2H_5)_{1,5}AlCl_{1,5}$ | $AlCl_3$ | ca. 40°C | $CH_3(CH_2)_2COC_2H_5$ | 95% |
| | desgl. | desgl. | desgl. | 0°C (Vergl.) | desgl. | 95% |
| | desgl. | $(n\text{-}C_6H_{13})AlCl_2$ (0,3 Mol) | – | ca. 40°C | $CH_3(CH_2)_2COC_6H_{13}$ | 88% |
| | 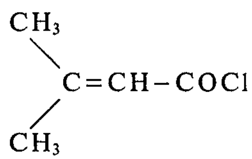 | $(CH_3)_{1,5}AlCl_{1,5}$ | $AlCl_3$ | ca. 40°C | 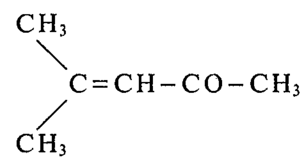 | 94% |
| | desgl. | desgl. | desgl. | 0°C (Vergl.) | desgl. | 93% |
| B) aromatisch: | 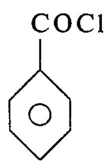 | $(CH_3)_{1,5}AlCl_{1,5}$ | $AlCl_3$ | ca. 40°C | 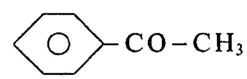 | 97% |
| | desgl. | desgl. | desgl. | 0°C (Vergl.) | desgl. | 82% |
| | desgl. | $(C_2H_5)_{1,5}AlCl_{1,5}$ | desgl. | ca. 40°C | 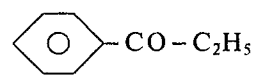 | 95% |
| | 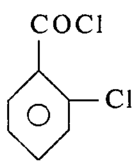 | $(CH_3)_{1,5}AlCl_{1,5}$ | $AlCl_3$ | ca. 40°C | 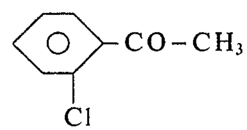 | 98% |
| | desgl. | desgl. | desgl. | 22–25°C (Vergl.) | desgl. | 95% |

Fortsetzung

| Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|
| 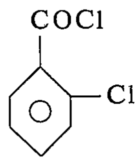 | $(CH_3)_{1,5}AlCl_{1,5}$ | $AlCl_3$ | 60°C/2 bar | 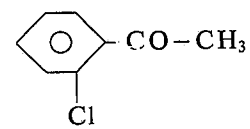 | 96% |
| desgl. | desgl. | desgl. | 0°C (Vergl.) | desgl. | 94% |
| desgl. | $(C_2H_5)_{1,5}AlCl_{1,5}$ | desgl. | ca. 40°C | 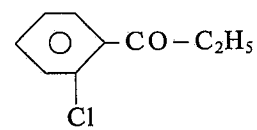 | 94% |
| 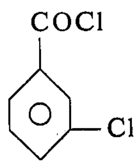 | $(CH_3)_{1,5}AlCl_{1,5}$ | $AlCi_3$ | ca. 40°C | 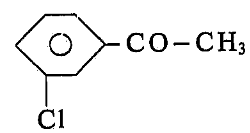 | 96% |
| desgl. | $CH_3AlCl_2$ (0,3 Mol) | – | ca. 40°C | desgl. | 95 |
| 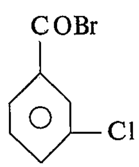 | $(CH_3)_2AlBr$ (0,15 Mol) | $AlBr_3$ (0,15 Mol) | ca. 40°C | desgl. | 96% |

Fortsetzung

| Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|
| 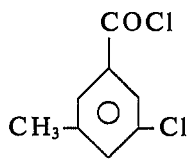 | $(C_2H_5)_{1,5}AlCl_{1,5}$ | $AlCl_3$ | ca. 40°C | 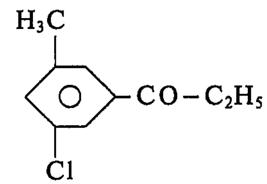 | 91% |
| 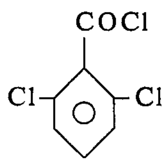 | $(CH_3)_{1,5}AlCl_{1,5}$ | $AlCl_3$ | ca. 40°C | 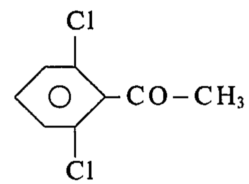 | 96% |
| 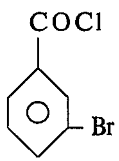 | $(C_2H_5)_{1,5}AlCl_{1,5}$ | $AlCl_3$ | ca. 40°C | 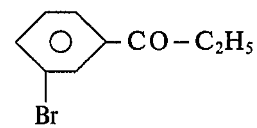 | 95% |
| 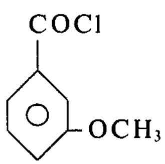 | $(CH_3)_{1,5}AlCl_{1,5}$ | $AlCl_3$ (0,4 Mol) | ca. 40°C | 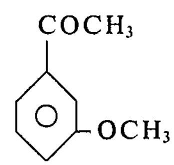 | 89% |

Fortsetzung

| Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|
| 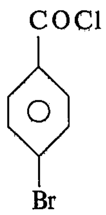 | $(CH_3)_{1,5}AlCl_{1,5}$ | $AlCl_3$ | ca. 40°C | 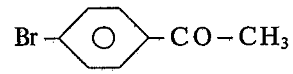 | 96% |
| desgl. | $(C_2H_5)_{1,5}AlCl_{1,5}$ | desgl. | ca. 40°C | 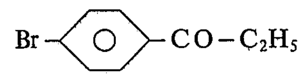 | 92% |
| 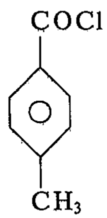 | $(CH_3)_{1,5}AlCl_{1,5}$ | $AlCl_3$ | ca. 40°C | 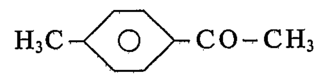 | 98% |
| desgl. | $(C_2H_5)_{1,5}AlCl_{1,5}$ | desgl. | ca. 40°C | 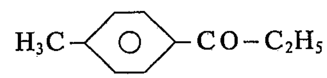 | 95% |
| 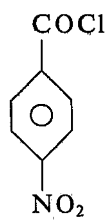 | $(CH_3)_{1,5}AlCl_{1,5}$ | $AlCl_3$ | ca. 40°C | 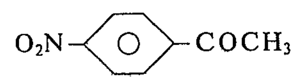 | 41% |

Fortsetzung

| | Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|---|
| | 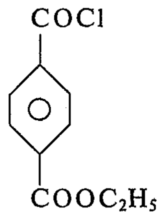 | $(CH_3)_{1,5}AlCl_{1,5}$ (0,21 Mol) | $AlCl_3$ (0,4 Mol) | ca. 40°C | 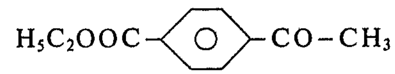 | 72% |
| | 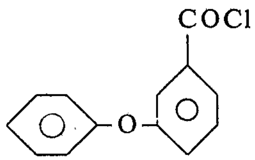 | $(CH_3)_{1,5}AlCl_{1,5}$ (0,3 Mol) | – | ca. 40°C | 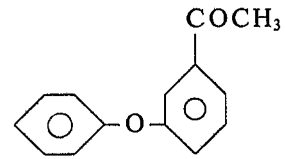 | 75% |
| C) arali-phatisch: | 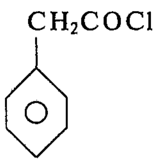 | $(CH_3)_{1,5}AlCl_{1,5}$ | $AlCl_3$ | ca. 40°C | 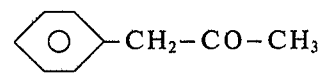 | 86% |
| | desgl. | $(C_2H_5)_{1,5}AlCl_{1,5}$ | desgl. | ca. 40°C | 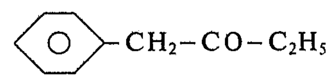 | 84% |
| | desgl. | desgl. | desgl. | 0°C (Vergl.) | desgl. | 80% |
| | 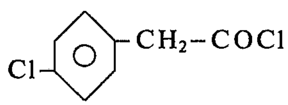 | $(CH_3)_{1,5}AlCl_{1,5}$ | $AlCl_3$ | ca. 40°C | 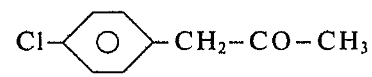 | 85% |
| | 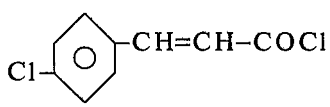 | $(CH_3)_{1,5}AlCl_{1,5}$ | $AlCl_3$ | ca. 40°C | 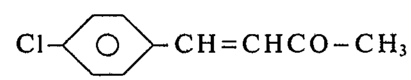 | 88% |

Fortsetzung

| | Ausgangs-Carbonsäure-halogenid | Al-Alkyl oder Alkyl-Al-Halogenid | Al-Halogenid | Temperatur | Reaktionsprodukt Keton | Ausbeute (% d. Th.) |
|---|---|---|---|---|---|---|
| D) hetero-cyclisch: | 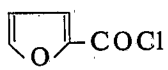 | $(CH_3)_{1,5}AlCl_{1,5}$ (0,21 Mol) | $AlCl_3$ (0,2–0,4 Mol) | ca. 40°C | 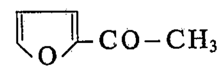 | 75% |
| | desgl. | desgl. | desgl. | 0°C (Vergl.) | desgl. | 0% |
| | 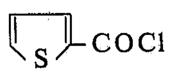 | $(CH_3)_{1,5}AlCl_{1,5}$ (0,21 Mol) | $AlCl_3$ (0,2–0,4 Mol) | ca. 40°C | 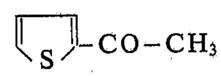 | 88% |
| | desgl. | desgl. | desgl. | 0°C (Vergl.) | desgl. | 15% |

**Patentansprüche**

1. Verfahren zur Herstellung von Ketonen durch Umsetzung von Carbonsäurehalogeniden mit Al-Alkylen oder Alkyl-Al-Halogeniden, gegebenenfalls in Gegenwart eines Al-Halogenids, in Methylenchlorid als Lösungsmittel und Zersetzung des entstehenden Ketonkomplexes in Wasser, dadurch gekennzeichnet, daß man die Umsetzung bei Temperaturen im Bereich von 30 bis 60°C, insbesondere etwa 40°C, gegebenenfalls unter Anwendung von Überdruck, durchführt.

2. Verfahren nach Anspruch 1, dadurch gekennzeichnet, daß man als Carbonsäurehalogenide Verbindungen der allgemeinen Formel

$R^1COX$

verwendet, worin

$R^1$ = a) gesättigter oder ungesättigter, verzweigter oder unverzweigter aliphatischer Rest, vorzugsweise mit 4 bis 20, insbesondere 4 bis 8 C-Atomen;
b) gegebenenfalls ein- oder mehrfach substituierter Aryl-, vorzugsweise Phenylrest;
c) ein Aralkyl-, vorzugsweise der Benzylrest, gegebenenfalls ebenfalls ein- oder mehrfach substituiert oder
d) ein heterocyclischer, vorzugsweise O- und/oder S-haltiger heterocyclischer Rest, insbesondere der Furyl- oder Thienylrest, und

X = Halogen, vorzugsweise Cl.

3. Verfahren nach Ansprüchen 1 und 2, dadurch gekennzeichnet, daß man als Al-Alkyle und Alkyl-Al-Halogenide Verbindungen der allgemeinen Formel

$R^2_nAlX_{3-n}$

verwendet, worin

$R^2$ = gesättigter, verzweigter oder unverzweigter Alkylrest, vorzugsweise mit 1 bis 12, insbesondere mit 1 bis 3 C-Atomen,
X = Halogen, vorzugsweise Cl und
n = 1, 1,5, 2 oder 3, vorzugsweise 1,5.

4. Verfahren nach Ansprüchen 1 bis 3, dadurch gekennzeichnet, daß man als Al-Halogenid ein Al-Halogenid mit dem gleichen Halogenrest wie das Alkyl-Al-Halogenid, vorzugsweise $AlCl_3$, verwendet.

5. Verfahren nach Ansprüchen 1 bis 4, dadurch gekennzeichnet, daß man pro Äquivalent Carbonsäurehalogenid

a) etwa 1 Mol Alkyl-Al-Dihalogenid $R^2AlX_2$,
b) etwa $^1/_2$ Mol Dialkyl-Al-Halogenid $R^2_2AlX$ + etwa $^1/_2$ Mol Al-Halogenid
c) etwa $^2/_3$ Mol Alkyl-Al-sesqui-Halogenid $R^2_{1,5}AlX_{1,5}$ + etwa $^1/_3$ Mol Al-Halogenid, oder
d) etwa $^1/_3$ Mol Trialkylaluminium $R^2_3Al$ + etwa $^2/_3$ Mol Al-Halogenid

verwendet.

6. Verfahren nach Ansprüchen 1 bis 5, dadurch gekennzeichnet, daß man die Umsetzung im Temperaturbereich von 30 bis etwa 40°C unter Normaldruck und im darüberliegenden Temperaturbereich unter autogenem (Über-)Druck durchführt.

**Claims**

1. Process for the manufacture of ketones by reacting carboxylic acid halides with Al-alkyls or alkyl-Al-halides optionally in the presence of an aluminum halide in $CH_2Cl_2$ as the solvent, and decomposing the ketone complex formed with water, characterized in carrying out the reaction at a temperature of from 30 to 60°C, in particular of about 40°C, optionally under an overpressure.

2. The process as claimed in claim 1, characterized in using as carboxylic acid halides compounds of the formula

$R^1COX$

in which
$R^1$ is

a) a saturated or unsaturated, branched or linear aliphatic radical, having preferably from 4 to 20, in particular from 4 to 8, carbon atoms:
b) aryl, preferably phenyl, optionally substituted once or several times,
c) aralkyl, preferably benzyl, optionally substituted once or several times or
d) a heterocyclic, preferably O- and/or S-containing radical, in particular furyl or thienyl, and

X is halogen, preferably chlorine.

3. The process as claimed in claims 1 and 2, characterized in using as Al-alkyl and alkyl-Al-halides compounds of the formula

$R^2_nAlX_{3-n}$

in which

$R_2$ is saturated branched or linear alkyl, having preferably from 1 to 12, in particular from 1 to 3, carbon atoms;
X is halogen, preferably chlorine and
n is 1, 1.5, 2 or 3, preferably 1.5.

4. The process as claimed in claims 1 to 3, characterized in using as Al-halide an Al-halide carrying the same halogen radical as the alkyl-Al-halide, preferably $AlCl_3$.

5. The process as claimed in claims 1 to 4, characterized in using per equivalent of carboxylic acid halide

a) about 1 mol of alkyl-Al-dihalide $R^2AlX_2$,
b) about $^1/_2$ mol of dialkyl-Al-halide $R^2_2AlX$ + about $^1/_2$ mol of Al-halide,
c) about $^2/_3$ mol of alkyl-Al-sesquihalide $R^2_{1.5}AlX_{1.5}$ + about $^1/_3$ mol of Al-halide or
d) about $^1/_3$ mol of trialkyl-Al $R^2_3Al$ + about $^2/_3$ mol of Al-halide.

6. The process as claimed in claims 1 to 5, characterized in carrying out the reaction at a temperature in the range from 30 to about 40°C under normal pressure, and at a temperature thereabove under autogenous (over-) pressure.

**Revendications**

1. Procédé de préparation de cétones par réaction d'halogénures d'acides carboxyliques avec des alkyl-aluminiums ou des halogénures d'alkyl-aluminiums, éventuellement en présence d'un halogénure d'aluminium, dans du chlorure de méthylène comme solvant, et décomposition dans de l'eau du complexe engendré par la cétone, procédé caractérisé en ce qu'on effectue la réaction à des températures situées dans l'intervalle de 30 à 60°C, plus particulièrement à environ 40°C, éventuellement en opérant sous pression.

2. Procédé selon la revendication 1, caractérisé en ce que les halogénures d'acides carboxyliques utilisés sont des composés répondant à la formule générale

$R^1COX$

dans laquelle

$R^1$ représente a) un radical aliphatique saturé ou insaturé, ramifié ou non ramifié, contenant de préférence de 4 à 20 atomes de carbone, plus particulièrement de 4 à 8,
b) un radical aryle, de préférence phényle, éventuellement mono- ou polysubstitué,
c) un radical aralkyle, de préférence phényle, qui peut aussi être mono- ou polysubstitué,
d) un radical hétérocyclique, de préférence un radical hétérocyclique contenant O- et/ou S-, plus spécialement un radical furyle ou thiényle,
et
X représente un halogène, de préférence Cl.

3. Procédé selon l'une des revendications 1 et 2, caractérisé en ce qu'on utilise, comme alkylaluminiums ou halogénures d'alkyl-aluminiums, des composés répondant à la formule générale

$R^2_nAlX_{3-n}$

dans laquelle

- $R^2$ représente un radical alkyle saturé, linéaire ou ramifié, contenant de préférence de 1 à 12 atomes de carbone, plus spécialement de 1 à 3,
- X représente un halogène, de préférence Cl, et
- n est égal à 1, à 1,5, à 2 ou à 3, de préférence à 1,5.

4. Procédé selon l'une quelconque des revendications 1 à 3, caractérisé en ce qu'on utilise un halogénure d'aluminium qui a le même halogène que l'halogénure d'alkyl-aluminium, de préférence $AlCl_3$.

5. Procédé selon l'une quelconque des revendications 1 à 4, caractérisé en ce qu'on utilise, par équivalent d'halogénure d'acide carboxylique:

a) environ 1 mole d'un dihalogénure d'alkylaluminium $R^2AlX_2$,
b) environ 1/2 mole d'un halogénure de dialkylaluminium $R^2_2AlX$ + environ 1/2 mole d'un halogénure d'aluminium,
c) environ 2/3 de mole d'un sesquihalogénure d'alkyl-aluminium $R^2_{1,5}AlX_{1,5}$ + environ 1/3 de mole d'un halogénure d'aluminium, ou
d) environ 1/3 de mole d'un trialkylaluminium $R^2_3Al$ + environ 2/3 de mole d'un halogénure d'aluminium.

6. Procédé selon l'une quelconque des revendications 1 à 5, caractérisé en ce qu'on effectue la réaction entre 30 et environ 40°C sous la pression normale, ou au-dessus sous la pression autogène quand on opère sous pression.